# EUROPEAN PATENT APPLICATION

(11) **EP 4 644 558 A1**
(43) Date of publication of application: **05.11.2025**
(21) Application number: 23912280.7
(22) Date of filing: 27.12.2023
(51) Int. Cl.: C12P 7/06

(54) **METHOD FOR PRODUCING ETHANOL FROM LIGNOCELLULOSE-BASED RAW MATERIAL**

(30) Priority: 28.12.2022 JP 2022212741
(71) Applicant: ENEOS Corporation, Chiyoda-ku Tokyo 100-8162 (JP)
(72) Inventor: FUKUDA, Akira, Tokyo 100-8162 (JP); KATO, Miyuki, Tokyo 100-8162 (JP); IDE, Kohei, Tokyo 100-8162 (JP); WASAI, Masafumi, Tokyo 100-8162 (JP); MUTAGUCHI, Kozue, Tokyo 100-8162 (JP)
(74) Representative: Müller-Boré & Partner Patentanwälte PartG mbB
(86) International application number: PCT/JP2023/047079
(87) International publication number: WO 2024/143503

(57) **Abstract**

The present invention provides a method for producing ethanol with an improved flow rate of permeation in membrane separation. More specifically, the present invention provides a method for producing ethanol from a lignocellulosic raw material, comprising a first step of performing saccharification and fermentation in a reaction system containing a lignocellulosic raw material, a saccharification enzyme, a yeast, and water, thereby producing ethanol; and a second step of treating, with a separation membrane, a first mixture containing the ethanol, water, the yeast, the saccharification enzyme, and a reaction residue of the lignocellulosic raw material, obtained in the first step, thereby obtaining a mixture containing the ethanol and water, and a second mixture containing the yeast, the saccharification enzyme, water, and the reaction residue of the lignocellulosic raw material; wherein the concentration of suspended solids in the first mixture is from 4 to 9.5% by mass.

## Description

### CROSS-REFERENCE TO RELATED APPLICATIONS

This application is based upon and claims the benefit of priority from Japanese Patent Application No. 2022-212741, filed on December 28, 2022; the entire contents of which are incorporated herein by reference.

### TECHNICAL FIELD

The present invention relates to a method for producing ethanol from a lignocellulosic raw material.

### BACKGROUND ART

A technology of producing saccharides from non-edible lignocellulosic raw materials is a technology extremely beneficial to the formation of a recycling-oriented society since it can produce alcohols that may be used as a gasoline substitute fuel, by using, as fermentation substrates for microorganisms, the saccharides produced, and also can prevent competition with food.

There has been known, as methods for producing, saccharides such as monosaccharides and oligosaccharides that serve as fermentation substrates from polysaccharides in lignocellulosic raw materials, an enzymatic saccharification methods using saccharification enzymes such as cellulolytic enzymes and microorganisms that produce saccharification enzymes.

Further, there are known, as a process in which an enzymatic saccharification method is applied for the production of ethanol from lignocellulose, processes referred to as hybrid hydrolysis and fermentation (multiple semi-parallel fermentation) and multiple parallel fermentation. In the case of hybrid hydrolysis and fermentation, a lignocellulosic raw material and a saccharification enzyme are added into one reaction tank and a saccharification reaction is performed therein, and then an ethanol-fermenting microorganism is added into the same reaction tank, and the enzymatic saccharification reaction and ethanol fermentation are performed therein. In the case of multiple parallel fermentation (also referred to as simultaneous saccharification and fermentation), an ethanol-fermenting microorganism is allowed to coexist with an above-described saccharification enzyme, and an enzymatic saccharification reaction and ethanol fermentation are simultaneously performed.

The production of ethanol from lignocellulosic raw materials has a problem that the production cost is high as compared with methods for ethanol production using starch, molasses and the like as raw materials. Therefore, there have been studied methods for efficient production of ethanol from lignocellulosic raw materials. However, the enzyme that is used in a method for ethanol production from a lignocellulosic raw material accounts for a large proportion of the production cost, and effective utilization of the enzyme used is necessary for the method to be put to practical use.

Patent Document 1 describes a method for manufacturing a sugar solution by hydrolyzing cellulose using a cellulase from a filamentous fungus as a saccharifying enzyme, the method comprising a step of adding the saccharifying enzyme to the cellulose to perform primary hydrolysis, followed by subjecting the primary hydrolysis product to solid-liquid separation into a primary sugar solution and solids; a step of adding water to the solids to perform secondary hydrolysis, followed by subjecting the secondary hydrolysis product to solid-liquid separation into a secondary sugar solution and a residue; and a step of filtering the primary sugar solution and/or the secondary sugar solution through an ultrafiltration membrane, thereby recovering the saccharifying enzyme from the non-permeate side and recovering a sugar solution from the permeate side. In Patent Document 1, it is described that according to this technology, this method has an effect of increasing the amount of production of the sugar, and an effect of increasing the amount of recovery of the enzyme.

As described in Patent Document 1, recovering of the used enzyme through a separation membrane and reusing of the recovered enzyme are considered as an effective method of enzyme utilization. However, as described in Non-Patent Document 1, it is known that during the membrane separation operation, suspended solids in the treated water block pores of the membrane or deposit on the membrane surface, leading to reduced membrane performance. Also in cases of filtering an ethanol fermentation solution through a separation membrane, there is a problem that the pore blockage in the membrane and deposition on the membrane surface caused by suspended solids result in a decreased flow rate of permeation, leading to decreased amounts of ethanol permeation and enzyme recovery per membrane area.

Therefore, there is still a need for efficient production of ethanol from lignocellulosic raw materials.

### RELATED ART DOCUMENTS

### Patent literature

Patent literature 1: WO 2011/115039 A
Non-patent literature 1: Membrane, 39 (4), 209-216 (2014)

### SUMMARY OF THE INVENTION

The present inventors have recently found that an improved flow rate of permeation through a separation membrane in a solid-liquid separation step is achieved by performing saccharification and fermentation in a reaction system containing a lignocellulosic raw material, a saccharification enzyme, a yeast, and water, such that a first mixture containing the ethanol, water, the saccharification enzyme, the yeast, and a reaction residue of the lignocellulosic raw material, obtained thereby, has a solid content concentration in a specified range. The present invention is based on these findings.

Accordingly, the present invention provides a method for producing ethanol from a lignocellulosic raw material with an improved flow rate of permeation in membrane separation.

The present invention includes the following [1] to [16].
[1] A method for producing ethanol from a lignocellulosic raw material, comprising:
   a first step of performing saccharification and fermentation in a reaction system containing a lignocellulosic raw material, a saccharification enzyme, a yeast, and water, thereby producing ethanol; and
   a second step of treating, with a separation membrane, a first mixture containing the ethanol, water, the yeast, the saccharification enzyme, and a reaction residue of the lignocellulosic raw material, obtained in the first step, thereby obtaining a mixture containing the ethanol and water, and a second mixture containing the yeast, the saccharification enzyme, water, and the reaction residue of the lignocellulosic raw material;
   wherein the concentration of suspended solids in the first mixture is from 4 to 9.5% by mass.
[2] The method for producing ethanol according to [1], further comprising a third step of feeding at least a portion of the second mixture to the reaction system.
[3] The method for producing ethanol according to [1] or [2], wherein the first step, the second step, and the third step are repeated.
[4] The method for producing ethanol according to any one of [1] to [3], wherein the separation membrane is an organic membrane.
[5] The method for producing ethanol according to any one of [1] to [4], wherein the separation membrane is an ultrafiltration membrane.
[6] The method for producing ethanol according to any one of [1] to [5], wherein the separation membrane is an organic membrane and an ultrafiltration membrane.
[7] The method for producing ethanol according to any one of [1] to [6], wherein the concentration of suspended solids in the first mixture is from 5 to 9.5% by mass.
[8] The method for producing ethanol according to any one of [1] to [7], wherein the concentration of suspended solids in the first mixture is from 5.5 to 9.5% by mass.
[9] The method for producing ethanol according to any one of [1] to [8], wherein the concentration of suspended solids in the first mixture is from 6.5 to 9.5% by mass.
[10] The method for producing ethanol according to any one of [1] to [9], wherein the concentration of suspended solids in the first mixture is from 7 to 9% by mass.
[11] The method for producing ethanol according to any one of [1] to [10], wherein the separation membrane has a form of a tubular membrane.
[12] The method for producing ethanol according to any one of [1] to [11], wherein the fermentation is a multiple parallel fermentation.
[13] A system for producing ethanol from a lignocellulosic raw material, comprising:
   a reaction tank allowing performing of saccharification and fermentation in a reaction system containing a lignocellulosic raw material, a saccharification enzyme, a yeast, and water, thereby producing ethanol; and
   a membrane separation apparatus comprising a separation membrane by which a first mixture containing the ethanol, water, the yeast, the saccharification enzyme, and a reaction residue of the lignocellulosic raw material, obtained in the reaction tank, is allowed to be separated into a mixture containing the ethanol and water, and a second mixture containing the yeast, the saccharification enzyme, water, and the reaction residue of the lignocellulosic raw material;
   wherein the concentration of suspended solids in the first mixture is from 4 to 9.5% by mass.
[14] The system for producing ethanol according to [13], comprising:
   a first step of performing saccharification and fermentation in a reaction system containing a lignocellulosic raw material, a saccharification enzyme, a yeast, and water, thereby producing ethanol; and
   a second step of treating, with the separation membrane, a first mixture containing the ethanol, water, the yeast, the saccharification enzyme, and a reaction residue of the lignocellulosic raw material, obtained in the first step, thereby obtaining a mixture containing the ethanol and water, and a second mixture containing the yeast, the saccharification enzyme, water, and the reaction residue of the lignocellulosic raw material.
[15] The system for producing ethanol according to [13] or [14], further comprising a third step of feeding at least a portion of the second mixture to the reaction tank.
[16] The system for producing ethanol according to any one of [13] to [15], further comprising a concentration apparatus for concentrating the mixture containing the ethanol and water that has been obtained in the membrane separation apparatus.

According to the present invention, an improved flow rate of permeation through a separation membrane in a solid-liquid separation step can be achieved by performing saccharification and fermentation in a reaction system containing a lignocellulosic raw material, a saccharification enzyme, a yeast, and water, such that a first mixture containing the ethanol, water, the yeast, the saccharification enzyme, and a reaction residue of the lignocellulosic raw material, obtained thereby, has a solid content concentration in a specified range. The present invention is advantageous in that an improved flow rate of permeation through the separation membrane allows an increased enzyme percentage on the second mixture side (the non-permeate side) containing the yeast, the saccharification enzyme, water, and a reaction residue of the lignocellulosic raw material, thereby leading to an increased enzyme recovery rate.

### BRIEF DESCRIPTION OF THE DRAWINGS

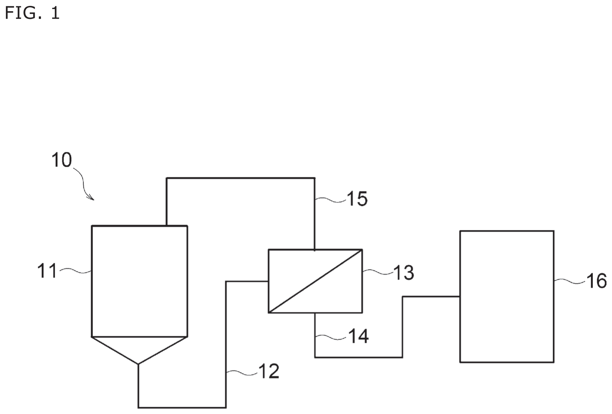

FIG. 1 is a diagram showing a schematic configuration of an ethanol production apparatus according to the system for producing ethanol of the present invention.

### DETAILED DESCRIPTION OF THE INVENTION

A method for producing ethanol from a lignocellulosic raw material of the present invention comprises a first step of performing saccharification and fermentation in a reaction system containing a lignocellulosic raw material, a saccharification enzyme, a yeast, and water, thereby producing ethanol; and a second step of treating, with a separation membrane, a first mixture containing the ethanol, water, the yeast, the saccharification enzyme, and a reaction residue of the lignocellulosic raw material, obtained in the first step, thereby obtaining a mixture containing the ethanol and water, and a second mixture containing the yeast, the saccharification enzyme, water, and the reaction residue of the lignocellulosic raw material, and has a characteristic that the concentration of suspended solids in the first mixture is from 4 to 9.5% by mass.

According to an embodiment of the present invention, the method further comprises a third step of feeding at least a portion of the second mixture to the reaction system.

According to a preferable embodiment of the present invention, the first step, the second step, and the third step are repeated.

Each step will be described in detail below.

### (First Step)

A first step is a step of performing saccharification and fermentation in a reaction system containing a lignocellulosic raw material, a saccharification enzyme, a yeast, and water, thereby producing ethanol. By this step, a first mixture can be obtained which contains the ethanol, water, the yeast, the saccharification enzyme, and a reaction residue of the lignocellulosic raw material. Such a first mixture may be an aqueous ethanol solution containing the yeast, the saccharification enzyme, and a reaction residue of the lignocellulosic raw material.

In the first step, saccharification and fermentation in the reaction system result in the saccharification of the lignocellulosic raw material by the saccharification enzyme (enzymatic saccharification reaction) and the conversion of the resultant saccharides into ethanol by fermentation with the yeast (ethanol fermentation). In the reaction system, a portion or the entire of the saccharification step may be performed simultaneously with the fermentation step. Therefore, the first step is a step of hybrid hydrolysis and fermentation or a step of multiple parallel fermentation, preferably a step of multiple parallel fermentation. Herein, in hybrid hydrolysis and fermentation, a saccharification reaction is performed using a lignocellulosic raw material and a saccharification enzyme in a reaction tank (saccharification step), and after that, a yeast is added into the same reaction tank, so that the enzymatic saccharification reaction and ethanol fermentation are simultaneously performed (saccharification-and-fermentation step). In multiple parallel fermentation, a yeast is allowed to coexist with an above-described saccharification enzyme, and an enzymatic saccharification reaction and ethanol fermentation are simultaneously performed (saccharification-and-fermentation step).

According to an embodiment of the present invention, in a step of multiple parallel fermentation, a reaction tank is first fed with a lignocellulosic raw material, a saccharification enzyme, and a yeast. In the present invention, a lignocellulosic raw material, a saccharification enzyme, and a yeast may be mixed beforehand, and then fed into a reaction tank, or alternatively may be each separately fed into a reaction tank. Preferably, a saccharification enzyme and a yeast are fed to a lignocellulosic raw material that has been fed beforehand into a reaction tank. The lignocellulosic raw material is saccharified by the saccharification enzyme and the resultant saccharides are converted into ethanol by fermentation with the yeast.

### (Lignocellulosic Raw Material)

A lignocellulosic raw material is pulp or a lignocellulose-containing raw material other than pulp. As the lignocellulosic raw material, one kind of lignocellulosic raw material may be used alone, or two or more kinds of lignocellulosic raw material may be mixed and used.

Examples of a pulp include wood pulp obtained from softwoods, hardwoods, forest residues, construction waste, and the like; non-wood pulps, such as cotton linter, cotton lint, hemp, straw, and bagasse; recycled pulp and deinked pulp made from used paper; and others. The method for manufacturing pulp are preferably ones by which lignin is highly removed, such as chemical pulp manufacturing methods, for example, ones with alkali extraction, alkali cooking, or the like. Among pulp manufactured by chemical pulp manufacturing methods, papermaking pulp is preferable in terms of availability. Examples of papermaking pulp include hardwood kraft pulp (e.g., leaf bleached kraft pulp (LBKP), leaf unbleached kraft pulp (LUKP), leaf oxygen bleached kraft pulp (LOKP)); softwood kraft pulp (e.g., needle bleached kraft pulp (NBKP), needle unbleached kraft pulp (NUKP), needle oxygen bleached kraft pulp (NOKP)); chemical pulp such as sulfite pulp (SP) and soda pulp (AP); and semichemical pulp such as semichemical pulp (SCP) and chemi-ground wood pulp (CGP). The pulp is preferably hardwood kraft pulp, softwood kraft pulp, sulfite pulp, or semichemical pulp, more preferably hardwood kraft pulp or softwood kraft pulp, and still more preferably leaf bleached kraft pulp (LBKP).

Examples of a lignocellulosic raw material other than pulp include, as wood-based materials, chips or barks derived from papermaking trees, forest residue, thinned wood, and others; sprouts generated from stumps of woody plants; *nokokuzu* or *ogakuzu* sawdust generated from sawmills and others; and pruned branches and leaves of street trees, and construction waste. It is possible to use, as the wood-based lignocellulosic raw material, ones derived from plants of the genus eucalyptus *(Eucalyptus),* plants of the genus salix (*Salix*), plants of the genus poplar, plants of the genus acacia (*Acacia*), plants of the genus cryptomeria (*Cryptomeria*), and others. Examples of a herbaceous raw material include agricultural wastes such as kenaf, rice straw, straw, corn cob and bagasse; residues and wastes of industrial crops such as oil crops and rubber (for example, empty fruit bunch (EFB), oil palm trunk (OPT), Oil Palm Frond (OPF), and meso cap fiber (MCF)); and herbaceous energy crops such as erianthus, miscanthus and napier grass. A lignocellulosic raw material other than pulp may also be biomass. Examples of a biomass that can be used in the present invention include wood-derived paper, used paper, pulp sludge, and the like. These biomasses can be used alone or in combination thereof. The biomass may be a dry solid material, a moisture-containing solid material, or a slurry.

According to an embodiment of the present invention, the concentration of the lignocellulosic raw material at the beginning of the reaction in the first mixture (fermentation liquid) in the reaction tank, in a step of multiple parallel fermentation, is preferably 5 to 30% by mass/volume, and more preferably 10 to 20% by mass/volume. Setting the concentration of the lignocellulosic raw material at 5% by mass/volume or more is advantageous in avoiding a problem that the concentration of the product is finally too low, leading to an increase in the cost of the concentration of ethanol. Setting the concentration of the lignocellulosic raw material at 30% by mass/volume or less is advantageous in avoiding a problem that it becomes difficult to stir the raw material as the concentration thereof increases, leading to lower productivity. According to another embodiment of the present invention, the concentration of the lignocellulosic raw material at the beginning of the reaction in the saccharification step, in a step of hybrid hydrolysis and fermentation, is similar as described above.

### (Saccharification Enzyme)

A saccharification enzyme is not particularly limited as long as it is a cellulolytic or hemicellulolytic enzyme. Examples of a cellulolytic enzyme include enzymes, generally referred to as so-called cellulase, which have cellobiohydrolase activity, endoglucanase activity, beta-glucosidase activity, and the like. For each of these cellulolytic enzymes, an enzyme having such an activity may be added in an appropriate amount. A commercially available cellulolytic enzyme agent may be used since many commercially available cellulolytic enzyme agents have the above-mentioned various cellulase activities as well as hemicellulase activity.

Examples of a commercially available cellulolytic enzyme agent include cellulolytic enzyme agents derived from the genus Trichoderma (*Trichoderma*), the genus Acremonium (*Acremonium*), the genus Aspergillus (*Aspergillus*), the genus Phanerochaete (*Phanerochaete*), the genus Trametes (*Trametes*), the genus Humicola (*Humicola*), the genus Bacillus (*Bacillus*), the genus Irpex (*Irpex*), and others. Examples of commercial products of such cellulolytic enzyme agents include, for example, Cellulosin AL8 (manufactured by HBI Enzymes Inc.), Cellic CTec2 (manufactured by Novozymes A/S), CELLULASE Y-NC (manufactured by Yakult Pharmaceutical Industry Co., Ltd.), and Meicelase (manufactured by Meiji Seika Pharma Co., Ltd.) (all trade names).

The saccharification enzymes to be used in the method can be used alone or in combination thereof, for example, in consideration of the properties of the cellulolytic enzyme agent.

The activity of the saccharification enzyme used in the present invention is defined in the following way.

A reaction is performed at 33°C for 4 hours in a total volume of 10 mL of a system in which 375 µL of an enzyme solution and 0.5 g (dry weight) of leaf bleached kraft pulp (LBKP) are added to 8 mL of an aqueous solution (pH 4.8) containing 1.5 mL of 1 M acetate buffer. After that, the reaction is stopped by heating at 95°C for 10 minutes. The reaction solution is then analyzed by high performance liquid chromatography (HPLC) (Prominence, manufactured by Shimadzu Corporation) using a differential refractive index (RI) detector, to determine the concentration of glucose. Based on the measurement results, the amount of enzyme protein that produces 1 µmol of glucose per minute is defined as 1 unit (U).

The measurement conditions for HPLC are as follows. Column: 80 Shodex SUGAR SP0810 (manufactured by Showa Denko K.K.)
Mobile phase: Ultrapure water
Flow rate: 0.8 mL/min
Temperature: 80°C

According to an embodiment of the present invention, the amount of the saccharification enzyme to be charged into the fermentation liquid in the reaction tank is not particularly limited and is preferably 50 to 500 U/L, and more preferably 100 to 300 U/L.

According to an embodiment of the present invention, the ratio of the amount of the saccharification enzyme charged to that of the lignocellulosic raw material in the fermentation liquid in the reaction tank (initial content (U) of the saccharification enzyme/initial content (kg) of the lignocellulosic raw material), in a step of multiple parallel fermentation, is preferably 500 to 5,000 U/kg, and more preferably 1,000 to 3,000 U/kg. According to another embodiment of the present invention, the ratio of the amount of the saccharification enzyme charged to that of the lignocellulosic raw material in the fermentation liquid in the reaction tank, in the saccharification-and-fermentation step in a step of hybrid hydrolysis and fermentation, is similar as described above.

### (Yeast)

A yeast is not particularly limited and is preferably capable of fermenting saccharides (hexoses, pentoses). Specifically, examples of such a yeast include yeasts of the genus *Saccharomyces* such as *Saccharomyces cerevisiae* (*Saccharomyces cerevisiae*); yeasts of the genus *Scheffersomyces* such as *Scheffersomyces stipitis* (*Scheffersomyces stipitis*) (changed from *Pichia stipitis* (*Pichia stipitis*)); yeasts of the genus *Candida* such as *Candida shihatae* (*Candida shihatae*); yeasts of the genus *Pachysolen* such as *Pachysolen tannophilus* (*Pachysolen tannophilus*); and others. Among these, a yeast is preferably a yeast belonging to the genus *Saccharomyces,* and more preferably *Saccharomyces cerevisiae.* Genetically modified yeasts can also be used which are prepared by using genetic recombination technology. As the genetically modified yeast, a yeast capable of simultaneously fermenting hexose and pentose sugars can be used without particular limitation. A preferable yeast prepared by using genetic recombination technology is ones having an improved capability of producing ethanol from xylose; more preferably ones into which a gene encoding for xylose reductase, a gene encoding for xylulokinase, a gene encoding for xylitol dehydrogenase, a gene encoding for transaldolase, a gene encoding for transketolase, and a gene encoding for alcohol dehydrogenase have been expressibly introduced; and still more preferably the yeast described in WO2016/060171 A. Here, such genes may be endogenous genes of an above-mentioned yeast.

According to an embodiment of the present invention, the pH of the fermentation liquid in the reaction tank in a step of multiple parallel fermentation is not particularly limited and is preferably maintained in a range of 3 to 10, and more preferably 4 to 8. According to another embodiment of the present invention, the pH of the fermentation liquid in the reaction tank in the saccharification-and-fermentation step in a step of hybrid hydrolysis and fermentation is similar as described above.

According to an embodiment of the present invention, the temperature of the fermentation liquid in the reaction tank in a step of multiple parallel fermentation is not particularly limited as long as it is within the optimum temperature range for the saccharification enzyme and/or the yeast, and is preferably from 20 to 40°C, and more preferably from 30 to 40°C. According to another embodiment of the present invention, the temperature of the fermentation liquid in the reaction tank in the saccharification-and-fermentation step in a step of hybrid hydrolysis and fermentation is similar as described above.

According to an embodiment of the present invention, the number of bacterial cells in the reaction tank in a step of multiple parallel fermentation is not particularly limited, and is preferably 10⁷ cells/mL or more, and more preferably 10⁸ cells/mL or more. The number of bacterial cells preferably ranges from 10⁷ to 10⁹ cells/mL, and more preferably from 10⁸ to 10⁹ cells/mL. According to another embodiment of the present invention, the number of bacterial cells in the reaction tank in the saccharification-and-fermentation step in a step of hybrid hydrolysis and fermentation is similar as described above.

According to an embodiment of the present invention, the number of bacterial cells to be inoculated in the reaction tank in a step of multiple parallel fermentation is not particularly limited, and is preferably 10⁶ cells/mL or more, and more preferably 10⁷ cells/mL or more. The number of bacterial cells preferably ranges from 10⁶ to 10⁸ cells/mL, and more preferably from 10⁷ to 10⁸ cells/mL. According to another embodiment of the present invention, the number of bacterial cells to be inoculated in the reaction tank in the saccharification-and-fermentation step in a step of hybrid hydrolysis and fermentation is similar as described above.

Suspended solids (hereinafter referred to as SS) in the first mixture (fermentation liquid) in the first step (preferably the step of multiple parallel fermentation) in the present invention refers to solid materials that exist in the fermentation liquid, and comprises the yeast, the saccharification enzyme, and a reaction residue of the lignocellulosic raw material.

The reaction residue of the lignocellulosic raw material in the first mixture may comprise, for example, lignin and the like.

In addition to the ethanol, water, the yeast, the saccharification enzyme, and a reaction residue of the lignocellulosic raw material, the first mixture may further comprise an unreacted raw material, glucose, xylose, mannose, and others. When these are in solid form in the first mixture (fermentation liquid), they are included in the suspended solids in the first mixture.

The method for measuring the concentration of suspended solids is not particularly limited. For example, the concentration of suspended solids can be measured using an integrating sphere type turbidimeter "PT-200" (Nittoseiko Analytech Co., Ltd.)

The solid concentration of suspended solids in the first mixture (fermentation liquid) is not particularly limited, and ranges from 4 to 9.5% by mass, preferably from 5 to 9.5% by mass, more preferably from 5.5 to 9.5% by mass, still more preferably from 6.5 to 9.5% by mass, and still more preferably from 7 to 9% by mass, from the viewpoint of improving the flow rate of permeation through the separation membrane in the solid-liquid separation step. The method for adjusting the concentration of suspended solids in these ranges is not particularly limited. The adjusting may be done, for example, by repeating the first step, the second step, and the third step.

The step of multiple parallel fermentation is preferably in a continuous system, and also may be in a semi-batch system or in a batch system. Here, the continuous system refers to, for example, a mode in which the feeding of the raw materials and the production of ethanol are continued.

In the step of multiple parallel fermentation, it is preferable to adjust the concentration of the lignocellulosic raw material in the fermentation liquid so as to be maintained within a predetermined range. The predetermined range is from 5 to 30% by mass/volume, and preferably from 10 to 20% by mass/volume. The adjustment is performed, for example, by feeding the lignocellulosic raw material into the reaction tank. As described below, when in the solid-liquid separation step, the second mixture after separation of the mixture containing the ethanol and water (also referred to as a suspended solid concentrated fermentation liquid) is recovered and transferred into the reaction tank, it is preferable that the total feeding rate of the suspended solid concentrated fermentation liquid and the lignocellulosic raw material into the reaction tank and the discharging rate of the fermentation liquid from the reaction tank are on the same level.

According to an embodiment of the present invention, in the step of multiple parallel fermentation, it is preferable to adjust the concentration of the saccharification enzyme in the fermentation liquid so as to be maintained within a predetermined range. The predetermined range is from 50 to 500 U/L, and preferably from 100 to 300 U/L. The adjustment is performed, for example, by feeding the saccharification enzyme into the reaction tank. Further, in the solid-liquid separation step, the adjustment may also be performed by recovering the second mixture (suspended solid concentrated fermentation liquid) after separation of the mixture containing the ethanol and water and then transferring it into the reaction tank, thereby feeding the recovered saccharification enzyme into the reaction tank.

### (Second Step)

A second step is a step of treating, with a separation membrane, a first mixture containing the ethanol, water, the yeast, the saccharification enzyme, and a reaction residue of the lignocellulosic raw material, obtained in the first step, thereby obtaining a mixture containing the ethanol and water (a mixture of the ethanol and water, an aqueous ethanol solution, or the like), and a second mixture containing the yeast, the saccharification enzyme, water, and the reaction residue of the lignocellulosic raw material. This step is a solid-liquid separation step.

According to a preferable embodiment of the present invention, the second step is a step in which a first mixture containing the ethanol, water, the yeast, the saccharification enzyme, and a reaction residue of the lignocellulosic raw material is removed from the reaction system, and a mixture containing the ethanol and water is separated from the first mixture through a separation membrane, thereby recovering the mixture containing the ethanol and water, and at the same time, a second mixture containing the yeast, the saccharification enzyme, water, and the reaction residue of the lignocellulosic raw material.

The second mixture may be a residue obtained by removing, from the first mixture, a mixture containing the ethanol and water, and the like. In addition to the yeast, the saccharification enzyme, water, and the reaction residue of the lignocellulosic raw material, the second mixture may further comprise an unreacted raw material, glucose, xylose, ethanol, and others.

More specifically, the fermentation liquid (first mixture) discharged from the reaction tank in the second step may be separated into a suspended solid concentrated fermentation liquid (second mixture) and a mixture containing the ethanol and water, for example, into a suspended solid concentrated fermentation liquid (second mixture) and an aqueous ethanol solution, with a membrane separation apparatus. The obtained mixture containing the ethanol and water can be further concentrated with a distillation treatment or using various separation membranes, for example, using a zeolite membrane, thereby allowing the production of high purity ethanol.

### (Separation Membrane)

The separation membrane used in the second step is not particularly limited, as long as it allows permeation therethrough of ethanol and prevention of the enzyme from passing therethrough. The separation membrane is preferably an organic membrane. Organic membranes made of a variety of materials can be used, which materials are specifically polyethylene (PE), polysulfone (PS), polypropylene (PP), polyolefin (PO), polyethersulfone (PES), polyacrylonitrile (PAN), polyvinylidene fluoride (PVDF), regenerated cellulose (RC), cellulose acetate (CA), ethylene tetrafluoride (PTEF), sulfonated polysulfone (P-PS), sulfonated polyethersulfone (P-PES), polyvinyl alcohol (PVAL), polymethyl methacrylate (PMMA), cellulose esters, and the like, with polysulfone being more preferable.

According to a preferable embodiment of the present invention, the separation membrane used in the second step is an ultrafiltration (UF) membrane or a Nanofiltration (NF) membrane, and more preferably an ultrafiltration membrane. The fractional molecular weight of a separation membrane preferably ranges from 10,000 Da (daltons) to 1,000,000 Da, and more preferably from 20,000 Da to 500,000 Da. According to another preferable embodiment of the present invention, the fractional molecular weight of a separation membrane preferably ranges from 0.001 microns (µm) to 0.5 microns, and more preferably from 0.005 microns to 0.1 microns. The separation membrane may a membrane separation apparatus comprising it.

According to an embodiment of the present invention, the membrane form of a separation membrane that can be used in the second step can be of a tubular membrane, a hollow fiber membrane, a flat membrane, a spiral-type membrane, or the like. Preferably, the membrane form of a separation membrane that can be used in the second step is of a tubular membrane.

According to an embodiment of the present invention, the difference of pressure between both sides of the separation membrane used in the second step (the difference between the pressures at the membrane inlet and at the membrane outlet on the non-permeate side) is settable as appropriate, depending on the kind of the separation membrane, and without particular limitation, preferably ranges from 0.001 to 1.0 MPa.

According to an embodiment of the present invention, the membrane-surface linear velocity (flow rate/membrane area) for the separation membrane used in the second step is not particularly limited, and preferably ranges from 0.01 to 5.0 m/s.

According to a preferable embodiment of the present invention, the concentration of suspended solids in the first mixture is in a specified range, thereby allowing an improved flow rate of permeation through the separation membrane. Here, the flow rate of permeation refers to an amount of permeation of a permeate per area of the membrane in a unit time. This embodiment is advantageous in that such an improved flow rate of permeation through the separation membrane allows an increased enzyme percentage on the side of the second mixture (at the side of the non-permeate) containing the yeast, the saccharification enzyme, water, and the reaction residue of the lignocellulosic raw material, and leads to an increased enzyme recovery rate. For example, in a case of a high flow rate of permeation through the separation membrane, if the percentage of the enzyme in the first mixture (fermentation liquid) is 20%, the flow rate at the separation membrane inlet (at the side of the first mixture) is 100 m³/h, the flow rate at the side of the permeate (mixture containing the ethanol and water) through the separation membrane is 50 m³/h, and the flow rate at the side of the non-permeate (second mixture; suspended solid concentrated fermentation liquid) is 50 m³/h, then the percentage of the enzyme in the permeate is 0%, and thus the percentage of the enzyme in the non-permeate will be 40%. In a case of a low flow rate of permeation through the separation membrane, if the percentage of the enzyme in the first mixture is 20%, the flow rate at the separation membrane inlet is 100 m³/h, the flow rate of the permeate through the separation membrane is 10 m³/h, and the flow rate of the non-permeate is 90 m³/h, then the percentage of the enzyme in the permeate is 0%, and thus the percentage of the enzyme in the non-permeate will be 22%.

According to a preferable embodiment of the present invention, the flow rate of permeation through the separation membrane is, for example, 33 L/(m²·h) or higher, preferably 40 L/(m²·h) or higher, more preferably 45 L/(m²·h) or higher, and still more preferably 50 L/(m²·h) or higher. The flow rate of permeation through the separation membrane is not particularly limited as long as the effects of present invention are achieved, and for example, 100 L/(m²·h) or lower. According to another preferable embodiment of the present invention, the flow rate of permeation through the separation membrane in the present invention is for example, from 33 to 100 L/(m²·h), preferably from 40 to 90 L/(m²·h), and more preferably from 50 to 80 L/(m²·h).

### (Third Step)

A third step is a step of feeding at least a portion of the second mixture to the reaction system. In other words, the third step can be said to be a step of transferring a portion or the entire of the suspended solid concentrated fermentation liquid (second mixture) that has been removed from the fermentation liquid (first mixture) to the reaction tank.

According to an embodiment of the present invention, the reaction system which is fed with the second mixture is a reaction system in the saccharification-and-fermentation step in multiple parallel fermentation, and may be a reaction system in the saccharification step or a reaction system in the saccharification-and-fermentation step in hybrid hydrolysis and fermentation. According to another embodiment of the present invention, the reaction system which is fed with the second mixture is, in multiple parallel fermentation, a reaction tank in which the saccharification-and-fermentation step is being performed, and in hybrid hydrolysis and fermentation, may be a reaction tank in which the saccharification step is being performed, or a reaction tank in which the saccharification-and-fermentation step is being performed.

### (Ethanol Concentration Step)

According to an embodiment of the present invention, the mixture containing the ethanol and water that has been obtained in the second step can be further concentrated with a distillation treatment or a rectification treatment, or using various separation membranes, for example, a zeolite membrane, thereby allowing the production of high purity ethanol. The mixture containing the ethanol and water may be transferred into the reaction tank after removing the ethanol.

According to an embodiment of the present invention, the water-containing ethanol which has been concentrated with a distillation treatment or a rectification treatment, or using various separation membranes may be dehydrated. The dehydrating of the water-containing ethanol is carried out using various methods, such as a water adsorption method using a water adsorbent, and an azeotropic method using water and a third solvent other than ethanol. By dehydrating the water-containing ethanol, the water is removed therefrom, so that anhydrous ethanol is obtained. The anhydrous ethanol is collected, and the water removed is subjected to wastewater treatment.

According to the method for producing ethanol of the present invention, ethanol can be efficiently produced from a lignocellulosic raw material. The ethanol which is obtained by the method for producing ethanol can be used suitably as, for example, ethanol for fuel, ethanol for industrial use, ethanol for food additives, and the like.

According to another aspect of the present invention, there is provided a system for producing ethanol from a lignocellulosic raw material. The system for producing ethanol of the present invention comprises a reaction tank allowing performing of saccharification and fermentation in a reaction system containing a lignocellulosic raw material, a saccharification enzyme, a yeast, and water, thereby producing ethanol; and a membrane separation apparatus comprising a separation membrane by which a first mixture containing the ethanol, water, the yeast, the saccharification enzyme, and a reaction residue of the lignocellulosic raw material, obtained in the reaction tank is allowed to be separated into a mixture containing the ethanol and water, and a second mixture containing the yeast, the saccharification enzyme, water, and the reaction residue of the lignocellulosic raw material, wherein the system has a characteristic that the concentration of suspended solids in the first mixture is from 4 to 9.5% by mass.

According to an embodiment of the present invention, the system for producing ethanol of the present invention preferably further comprises a concentration apparatus for concentrating the mixture containing the ethanol and water that has been obtained in the membrane separation apparatus.

FIG. 1 is a diagram showing a schematic configuration of an ethanol production apparatus according to the system for producing ethanol of the present invention. In the drawing used in the following description, for the sake of convenience, essential parts may be enlarged in order to make it easier to understand the features of the present invention, and each of the components is not necessarily the same as the actual one in dimension, ratio, and others. Further, only essential parts are depicted, and there are not shown facilities for feeding the reaction tank with a lignocellulosic raw material, a yeast, and others, a pre-treatment apparatus, and the like, which are placed in front of the reaction tank; analyzers associated with the reaction tank and the membrane separation apparatus; a dehydration facility following the concentration apparatus, and other.

In the production apparatus 10 of the present invention, a reaction tank 11 and a membrane separation apparatus 13 are placed and connected via a pipeline 12. In this context, a first mixture obtained in the reaction tank 11 is transferred to the membrane separation apparatus 13 through the pipeline 12. The mixture containing ethanol and water which has been separated through the separation membrane in the membrane separation apparatus 13 is transferred to a concentration apparatus 16 through a pipeline 14, and the second mixture which has been separated through the separation membrane in the membrane separation apparatus 13 is transferred into the reaction tank 11 through a pipeline 15. A pre-treatment apparatus, or the like, may be placed in front of the reaction tank 11. An apparatus following the membrane separation apparatus 13 can be chosen as appropriate, depending on the use of the fermentation liquid. For example, a concentration apparatus 16 for performing a distillation treatment may be placed.

According to a preferable embodiment of the present invention, the system for producing ethanol of the present invention comprises a first step of performing saccharification and fermentation in a reaction system containing a lignocellulosic raw material, a saccharification enzyme, a yeast, and water, thereby producing ethanol; and a second step of treating, with a separation membrane, a first mixture containing the ethanol, water, the yeast, the saccharification enzyme, and a reaction residue of the lignocellulosic raw material, obtained in the first step, thereby obtaining a mixture containing the ethanol and water, and a second mixture containing the yeast, the saccharification enzyme, water, and the reaction residue of the lignocellulosic raw material, and preferably comprises a third step of feeding at least a portion of the second mixture to the reaction tank.

According to a preferable embodiment of the present invention, the system for producing ethanol of the present invention comprises performing, in the reaction tank 11, saccharification and fermentation in a reaction system containing a lignocellulosic raw material, a saccharification enzyme, a yeast, and water, thereby obtaining a first mixture containing the ethanol, water, the yeast, the saccharification enzyme, and a reaction residue of the lignocellulosic raw material. The first mixture obtained is transferred to the membrane separation apparatus 13 comprising a separation membrane, and treated with the separation membrane, thereby obtaining a mixture containing the ethanol and water, and a second mixture containing the yeast, the saccharification enzyme, water, and the reaction residue of the lignocellulosic raw material. The obtained mixture containing the ethanol and water can be transferred to the concentration apparatus 16 and concentrated therein. Into the reaction tank 11 can be transferred at least a portion of the second mixture which has been separated from the first mixture. For example, the obtaining of the first mixture in the reaction tank 11, the separating of the first mixture into the mixture containing the ethanol and water, and the second mixture, in the membrane separation apparatus 13 comprising a separation membrane, and the transferring of at least a portion of the second mixture into the reaction tank 11, as described above, can be repeated, thereby allowing the concentration of suspended solids in the first mixture to be adjusted to 4 to 9.5% by mass.

According to another embodiment of the present invention, the system for producing ethanol of the present invention is preferably used in a continuous system.

Aspects of the above-mentioned system for producing ethanol can be performed in accordance with the description of the method for producing ethanol of the present invention.

### EXAMPLES

The present invention will be described in more detail by way of the following Example, but the present invention is not limited thereto. Unless otherwise specified, the units and measurement methods described in the present specification are based on the Japanese Industrial Standards (JIS).

### Method for Measuring SS Concentration

The SS concentration in the first mixture (evaluation liquid) in the Example was measured using an integrating sphere type turbidimeter (PT-200 with a 10-mm cell, Nittoseiko Analytech Co., Ltd.).

### Method for Counting Number of Cells

Cells of a yeast *Saccharomyces cerevisiae* used in the Example were observed under an optical microscope (at a magnification of 400 times), and the number of bacterial cells was counted using a Thoma hemocytometer.

### Method for Measuring Flow Rate of Permeation

The flow rate of permeation in the Example was calculated from the amount of permeation in a unit time and the area of a membrane by receiving a permeate in a container and measuring its volume.

### Example 1: Studies on the relationship between SS concentration and flow rate of permeation

For a constant amount of permeation (amount of production) of ethanol in membrane separation, it is necessary to understand the relationship between SS concentration and flow rate of permeation. In the Example, evaluation liquids with various SS concentrations were prepared, and each of these liquids was subjected to permeation through a membrane, thereby measuring the flow rate of permeation.

(1) Into a 150 L culture tank (reaction tank) were charged 5% by mass/volume (on basis of dry weight) of pulp (leaf bleached kraft pulp (LBKP)), 19771 U of a saccharification enzyme, 0.22% by mass/volume of urea, 1% by mass/volume of CSL (corn steep liquor), and an antibacterial agent in an amount giving a concentration of 10 ppm, and then a yeast *Saccharomyces cerevisiae* was inoculated in an amount giving a final concentration of 1.0 x 10⁷ cells/mL. The volume of liquid was adjusted with sterile water to give a reaction weight of 142.5 kg. After that, the culture tank was controlled so as to become stabilized at a temperature of 33°C, at a stirring rate of 72 rpm, and at a pH of 4.8 (by controlling with sodium hydroxide and sulfuric acid), and the reaction was performed for 24 hours. After 24-hour reaction, the pulp was charged in an amount giving a final concentration of 10% by mass/volume (on basis of dry weight) (corresponding to 7.5 kg), to reach a total weight of 150 kg. Then, the reaction was performed for an additional 24 hours to generate an evaluation liquid, followed by measurement of the SS concentration therein. (2) After the reaction, the cultured liquid was left to stand for a period of 72 to 96 hours, and subjected to solid-liquid separation within the culture tank. (3) 75 L of the supernatant was discarded, and an additional evaluation liquid was prepared by adding, to the remaining evaluation liquid, 5% by mass/volume (on basis of dry weight) of the pulp, 9885 U of the saccharification enzyme, 0.11% by mass/volume of urea, 0.5% by mass/volume of CSL, and an antibacterial agent in an amount giving a concentration of 5 ppm, and sterile water was added to reach a total weight of 150 kg. Then, the reaction was performed again for 24 hours, followed by measurement of the SS concentration in the evaluation liquid. The steps (2) and (3) described above were repeated, thereby preparing eight additional evaluation liquids having an SS concentration of 1, 6, 7, 8, 9, 10, 11, and 12% by mass, which were each used for membrane evaluation.

The conditions under which the evaluation liquids were prepared are shown below:

**[Table 1]**

| Item | Value |
|---|---|
| Temperature, °C | 33 |
| Stirring rate, rpm | 72 |
| pH | 4.8 |
| Volume of evaluation liquid, L | 150 |
| Reaction time, hours | 48 |

The composition of the evaluation liquids is shown below:

**[Table 2]**

| Item | Value |
|---|---|
| Pulp, % by mass/volume (on basis of dry weight) | 10 |
| Amount of saccharification en zyme added, U | 19771 |
| Urea, % by mass/volume | 0.22 |
| CSL (corn steep liquor), % by mass/volume | 1 |
| Anti-bacterial agent, ppm | 10 |

### Calculation of the flow rate of permeation at the respective SS concentrations

The membrane evaluation was performed as follows. A membrane module (manufactured by DAISEN MEMBRANE SYSTEMS Ltd., TU-30100-P18A, and comprising a UF membrane made of polysulfone, which is a tubular membrane with a fractional molecular weight of 100,000 Da) was connected to the 150 L culture tank. The liquid was circulated while a mixture (permeate) containing the ethanol and water, and a suspended solid concentrated fermentation liquid (non-permeate) were being returned into the 150 L culture tank, under conditions where the pressure difference between both sides of the membrane was 0.4 MPa and the membrane-surface linear velocity was 2.0 m/s. The flow rate of permeation (L/(m²·h)) of the mixture containing the ethanol and water was measured over time, to measure the value of a flow rate of permeation at the time when there were no changes over time in the flow rate of permeation.

The results of calculation of the flow rate of permeation at the respective SS concentrations are shown below.

**[Table 3]**

| | Test area | | | | | | | |
|---|---|---|---|---|---|---|---|---|
| | 1 | 2 | 3 | 4 | 5 | 6 | 7 | 8 |
| SS concentration (% by mass) | 1 | 6 | 7 | 8 | 9 | 10 | 11 | 12 |
| Flow rate of permeation (L/(m²·h)) | 32.4 | 42.1 | 51.2 | 50.1 | 50.5 | 24.9 | 19.5 | 15.9 |

From Table 3, it is ascertained that SS concentrations higher than 1% by mass and lower 10% by mass lead to the increase in the flow rate of permeation. Furthermore, SS concentrations between 7% and 9% by mass exhibit the highest flow rates of permeation.

### DESCRIPTION OF REFERENCE SIGNS

10: Production apparatus
11: Reaction tank
12, 14, 15: Pipelines
13: Membrane separation apparatus
16: Concentration apparatus

## Claims

1. A method for producing ethanol from a lignocellulosic raw material, comprising:
a first step of performing saccharification and fermentation in a reaction system containing a lignocellulosic raw material, a saccharification enzyme, a yeast, and water, thereby producing ethanol; and
a second step of treating, with a separation membrane, a first mixture containing the ethanol, water, the yeast, the saccharification enzyme, and a reaction residue of the lignocellulosic raw material, obtained in the first step, thereby obtaining a mixture containing the ethanol and water, and a second mixture containing the yeast, the saccharification enzyme, water, and the reaction residue of the lignocellulosic raw material;
wherein the concentration of suspended solids in the first mixture is from 4 to 9.5% by mass.

2. The method for producing ethanol according to claim 1, further comprising a third step of feeding at least a portion of the second mixture to the reaction system.

3. The method for producing ethanol according to claim 1 or 2, wherein the first step, the second step, and the third step are repeated.

4. The method for producing ethanol according to any one of claims 1 to 3, wherein the separation membrane is an organic membrane.

5. The method for producing ethanol according to any one of claims 1 to 4, wherein the separation membrane is an ultrafiltration membrane.

6. The method for producing ethanol according to any one of claims 1 to 5, wherein the separation membrane is an organic membrane and an ultrafiltration membrane.

7. The method for producing ethanol according to any one of claims 1 to 6, wherein the concentration of suspended solids in the first mixture is from 5 to 9.5% by mass.

8. The method for producing ethanol according to any one of claims 1 to 7, wherein the concentration of suspended solids in the first mixture is from 5.5 to 9.5% by mass.

9. The method for producing ethanol according to any one of claims 1 to 8, wherein the concentration of suspended solids in the first mixture is from 6.5 to 9.5% by mass.

10. The method for producing ethanol according to any one of claims 1 to 9, wherein the concentration of suspended solids in the first mixture is from 7 to 9% by mass.

11. A system for producing ethanol from a lignocellulosic raw material, comprising:
a reaction tank allowing performing of saccharification and fermentation in a reaction system containing a lignocellulosic raw material, a saccharification enzyme, a yeast, and water, thereby producing ethanol; and
a membrane separation apparatus comprising a separation membrane by which a first mixture containing the ethanol, water, the yeast, the saccharification enzyme, and a reaction residue of the lignocellulosic raw material, obtained in the reaction tank, is allowed to be separated into a mixture containing the ethanol and water, and a second mixture containing the yeast, the saccharification enzyme, water, and the reaction residue of the lignocellulosic raw material;
wherein the concentration of suspended solids in the first mixture is from 4 to 9.5% by mass.

12. The system for producing ethanol according to claim 11, comprising:
a first step of performing saccharification and fermentation in a reaction system containing a lignocellulosic raw material, a saccharification enzyme, a yeast, and water, thereby producing ethanol; and
a second step of treating, with the separation membrane, a first mixture containing the ethanol, water, the yeast, the saccharification enzyme, and a reaction residue of the lignocellulosic raw material, obtained in the first step, thereby obtaining a mixture containing the ethanol and water, and a second mixture containing the yeast, the saccharification enzyme, water, and the reaction residue of the lignocellulosic raw material.

13. The system for producing ethanol according to claim 11 or 12, further comprising a third step of feeding at least a portion of the second mixture to the reaction tank.

14. The system for producing ethanol according to any one of claims 11 to 13, further comprising a concentration apparatus for concentrating the mixture containing the ethanol and water that has been obtained in the membrane separation apparatus.
